# EUROPEAN PATENT APPLICATION

(11) **EP 1 674 089 A1**
(43) Date of publication of application: **28.06.2006**
(21) Application number: 04788019.0
(22) Date of filing: 22.09.2004
(51) Int. Cl.: A61K 31/19, A61K 31/215, A61P 3/10, A61P 5/50

(54) **ACCELERATOR FOR INITIAL INSULIN SECRETION**

(30) Priority: 22.09.2003 US 503893 P; 22.09.2003 US 503892 P; 26.12.2003 JP 2003434627; 27.02.2004 JP 2004055440; 27.02.2004 JP 2004055439; 01.03.2004 JP 2004056876; 21.04.2004 JP 2004126091
(71) Applicant: Use-Techno Corporation, Fukuchiyama-shi, Kyoto 6200055 (JP)
(72) Inventor: MATSUYAMA, Futoshi, 6208502 (JP); SEINO, Yutaka, 6610003 (JP); MIURA, Toshihiro, 5100206 (JP); FUJITA, Takeshi, 6650807 (JP); KANEKO, Tetsuo, Kure-shi, Hiroshima 737-0112 (JP); FUKUSHIMA, Mitsuo, Kobe-shi Hyogo 657-0061 (JP)
(74) Representative: Ouzman, Beverley Nicola Claire
(86) International application number: PCT/JP2004/013836
(87) International publication number: WO 2005/027891

(57) **Abstract**

The present invention provides an early insulin secretion stimulator consisting of a triterpene represented by the following general formula (1) and/or a triterpene represented by the following general formula (2). [where R¹ represents COOH, etc., R¹¹ and R¹² represent CH₂OH, etc., X¹ represents hydrogen or OH and X¹¹, X¹², X²¹ and X²² represent OH, etc.]

## Description

### Technical Field

The present invention relates to an early insulin secretion stimulator.

### Background Art

Banaba (*Lagerstfoemia speciosa Linn.* or *Pers.)* is a plant of *Lythraceae* which is found widely in Southeast Asian countries such as the Philippines, India, Malaysia, Southern China and Australia. Patent Document 1 proposes an antidiabetic agent composed mainly of banaba extract obtained from banaba leaves using hot water or an organic solvent, and the antidiabetic effect thereof has been confirmed in animal experiments on diabetic mice.
Patent Document 1: Japanese Patent Application Laid-Open No. 5-310587

### Disclosure of the Invention

### Problem to be Solved by the Invention

For treatment of diabetes it is ideal to achieve rapid secretion of insulin immediately after meals while avoiding oversecretion of insulin in the absence of blood glucose increase. However, the current antidiabetic agents, or synthetic drugs for diabetes treatment such as sulfonylurea agents, biguanide agents, thiazolidine derivatives and phenylalanine derivatives have not been able to readily achieve such ideal blood glucose increase suppression and insulin secretion control.

These antidiabetic agents and synthetic drugs, while successfully lowering blood glucose levels, tend to cause hypoglycemia or can provoke insulin resistance (reduced insulin sensitivity) and in some cases may have side effects on the liver, while exhaustion of the pancreas, an insulin secreting organ, has been a particular unavoidable problem.

It is an object of the present invention to provide an early insulin secretion stimulator with low side effects, which rapidly stimulates early insulin secretion and suppresses blood glucose increase only at mealtimes, and is therefore able to exhibit ideal blood glucose increase suppression and insulin secretion control.

### Means for Solving the Problem

In order to achieve the aforementioned object, the present invention provides early insulin secretion stimulators according to the following (i) to (x).
(i) An early insulin secretion stimulator consisting of a triterpene represented by the following general formula (1) and/or a triterpene represented by the following general formula (2). 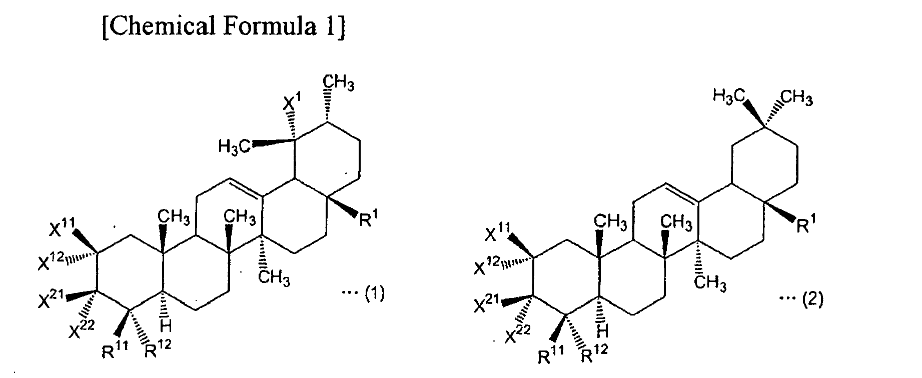 [where R¹ represents COOH, CHO, CH₃ or CH₂OH, R¹¹ and R¹² each independently represent CH₃, CH₂OH or COOH, X¹ represents hydrogen or OH, and X¹¹, X¹², X²¹ and X²² each independently represent hydrogen, OH or acyl, with the proviso that two among X¹¹, X¹², X²¹ and X²² must be hydrogen and the other two must be OH or acyl, X¹¹ and X¹² are not simultaneously OH or acyl, and X¹¹ and X¹² or X²¹ and X²² may together form an =O group.]
(ii) The early insulin secretion stimulator according to (i), wherein R¹ is COOH.
(iii) The early insulin secretion stimulator according to (i) or (ii), wherein at least one of R¹¹ and R¹² is CH₂OH.
(iv) The early insulin secretion stimulator according to any one of (i) to (iii), wherein X¹ is OH.
(v) The early insulin secretion stimulator according to any one of (i) to (iv), wherein at least one OH group of X¹¹, X¹², X²¹ and X²² is esterified. The number of carbon atoms of the esterification agent in this case is preferably 1-12 and more preferably 1-6.
(vi) The early insulin secretion stimulator according to any one of (i) to (iv), wherein at least one OH group of X¹¹, X¹², X²¹ and X²² is etherified. The number of carbon atoms of the etherification agent in this case is preferably 1-12 and more preferably 1-6.
(vii) The early insulin secretion stimulator according to any one of (i) to (iv), wherein two from among X¹¹, X¹², X²¹ and X²² are the same acyl group.
(viii) The early insulin secretion stimulator according to any one of (i) to (vii), wherein the acyl group is a group represented by R²CO- (with the proviso that R² is an alkyl group having 1-17 carbon atoms or a substituted or unsubstituted aryl group). As examples of groups represented by R²CO- there may be mentioned acetyl, propionyl, butyryl, valeryl, palmitoyl, stearoyl, oleoyl, benzoyl, toluoyl, salicyloyl, cinnamoyl, naphthoyl, phthaloyl and furoyl.
(ix) The early insulin secretion stimulator according to any one of (i) to (viii), wherein the acyl group is acetyl.
(x) The early insulin secretion stimulator according to any one of (i) to (ix), which is glucose-dependent.

### Effects of the Invention

According to the present invention there is provided an early insulin secretion stimulator having effects of inhibiting blood glucose increase, improving insulin resistance, preventing obesity and suppressing triglycerides by inducing rapid secretion of insulin immediately after meals without inducing excessive secretion of insulin in the absence of blood glucose increase. That is, there is provided an early insulin secretion stimulator which stimulates early secretion of insulin to lower postprandial blood glucose levels while preventing excessive insulin secretion.

### Brief Description of the Drawings

Fig. 1 is a graph showing changes in blood insulin levels during a glucose tolerance test, wherein (a) is a case with administration of corosolic acid and (b) is a case with administration of a placebo.
Fig. 2 is a graph showing blood glucose level changes.
Fig. 3 is a graph showing blood glucose level changes.
Fig. 4 is a graph showing blood glucose level changes.
Fig. 5 is a graph showing blood glucose level changes.

### Best Modes for Carrying Out the Invention

Triterpenes which may be used as the early insulin secretion stimulator according to the present invention include the following exemplary compounds 1) to 100).

The following compounds may be mentioned as ursane-type pentacyclic triterpenes.
1) Desfontainic acid
2) 2,19α-Dihydroxy-3-oxo-1,12-ursadien-28-oic acid
3) 2x,20β-Dihydroxy-3-oxo-12-ursen-28-oic acid
4) 2α,3α-Dihydroxy-12,20(30)-ursadien-28-oic acid
5) 2α,3β-Dihydroxy-12,20(30)-ursadien-28-oic acid
6) 2β,3β-Dihydroxy-12-ursen-23-oic acid
7) 2α,3α-Dihydroxy-12-ursen-28-oic acid
8) 1α,2α,3β,19α,23-Pentahydroxy-12-ursen-28-oic acid
9) 2α,3β,7α,19α,23-Pentahydroxy-12-ursen-28-oic acid
10) 1β,2α,3α,19α-Tetrahydroxy-12-ursen-28-oic acid
11) 1β,2α,3β,19α-Tetrahydroxy-12-ursen-28-oic acid
12) 1β,2β,3β,19α-Tetrahydroxy-12-ursen-28-oic acid
13) 2α,3β,6β,19α-Tetrahydroxy-12-ursen-28-oic acid
14) 2α,3β,6β,23-Tetrahydroxy-12-ursen-28-oic acid
15) 2α,3β,7α,19α-Tetrahydroxy-12-ursen-28-oic acid
16) 2α,3α,7β,19α-Tetrahydroxy-12-ursen-28-oic acid
17) 2α,3β,13β,23-Tetrahydroxy-11-ursen-28-oic acid
18) 2α,3α,19α,23-Tetrahydroxy-12-ursen-28-oic acid
19) 2α,3β,19α,23-Tetrahydroxy-12-ursen-28-oic acid
20) 2α,3α,19α,24-Tetrahydroxy-12-ursen-28-oic acid
21) 2α,3β,19α,24-Tetrahydroxy-12-ursen-28-oic acid
22) 2α,3β,23-Trihydroxy-11-oxo-12-ursen-28-oic acid
23) 2α,3β,24-Trihydroxy-12,20(30)-ursadien-28-oic acid
24) 2α,3β,27-Trihydroxy-28-ursanoic acid
25) 2α,3β,19α-Trihydroxy-12-ursene-23,28-dioic acid
26) 2α,3β,19α-Trihydroxy-12-ursene-24,28-dioic acid
27) 1β,2β,3β-Trihydroxy-12-ursen-23-oic acid
28) 2α,3β,6β-Trihydroxy-12-ursen-28-oic acid
29) 2α,3α,19α-Trihydroxy-12-ursen-28-oic acid
30) 2α,3β,19α-Trihydroxy-12-ursen-28-oic acid
31) 2α,3α,23-Trihydroxy-12-ursen-28-oic acid
32) 2α,3β,23-Trihydroxy-12-ursen-28-oic acid
33) 2α,3α,24-Trihydroxy-12-ursen-28-oic acid
34) 2α,3β,24-Trihydroxy-12-ursen-28-oic acid
35) 2α,3β,27-Ursanetriol
36) 12-Ursene-1β,2α,3β,11α,20β-pentol
37) 12-Ursene-1β,2α,3β,11α-tetrol
38) 12-Ursene-2α,3β,11α,20β-tetrol
39) 12-Ursene-2α,3β,11α-triol
40) 12-Ursene-2α,3β,28-triol

The following compounds may be mentioned as active oleanane-type pentacyclic triterpenes.
41) 2α,3β-Dihydroxy-12,18-oleanadiene-24,28-dioic acid
42) 2α,3β-Dihydraxy-12-oleanene-23,28-dioic acid
43) 2β,3β-Dihydroxy-12-oleanene-23,28-dioic acid
44) 2β,3β-Dihydroxy-12-oleanene-28,30-dioic acid
45) 2β,3β-Dihydroxy-12-oleanen-23-oic acid
46) 2β,3β-Dihydroxy-12-oleanen-28-oic acid
47) 2α,3α-Dihydroxy-12-oleanen-28-oic acid
48) 2α,3β-Dihydroxy-12-oleanen-28-oic acid
49) 2α,3β-Dihydroxy-13(18)-oleanen-28-oic acid
50) 12β,13β-Epoxy-2α,3β,21β,22β-tetrahydroxy-30-oleananoic acid
51) 13,28-Epoxy-2α,3β,16α,22β-tetrahydroxy-30-oleananoic acid
52) 13β,28-Epoxy-2α,3β,16α,22β-tetrahydroxy-30-oleananoic acid
53) 12-Oleanene-2α,3α-diol
54) 12-Oleanene-2α,3β-diol
55) 13(18)-Oleanene-2α,3α-diol
56) 13(18)-Oleanene-2β,3β-diol
57) 18-Oleanene-2α,3β-diol
58) 18-Oleanene-2α,3α-diol
59) 12-Oleanene-2α,3β,16β,21β,22α,28-hexol
60) 12-Oleanene-1β,2α,3β,11α-tetrol
61) 12-Oleanene-2β,3β,23,28-tetrol
62) 12-Oleanene-2α,3β,11α-triol
63) 12-Oleanene-2β,3β,28-triol
64) 12- Oleanene-2α,3β,23-triol
65) 13(18)-Oleanene-2α,3β,11α-triol
66) 2β,3β,6β,16α,23-Pentahydroxy-12-oleanen-28-oic acid
67) 2β,3β,16β,21β,23-Pentahydroxy-12-oleanen-28-oic acid
68) 2β,3β,16α,23,24-Pentahydroxy-12-oleanen-28-oic acid
69) 2β,3β,13β,16α-Tetrahydroxy-23,28-oleananedioic acid
70) 2β,3β,16β,21β-Tetrahydroxy-12-oleanen-24,28-dioic acid
71) 2β,3β,16α,23-Tetrahydroxy-12-oleanen-24,28-dioic acid
72) 2β,3β,22β,27-Tetrahydroxy-12-oleanen-23,28-dioic acid
73) 2α,3β,6β,23-Tetrahydroxy-12-oleanen-28-oic acid
74) 2β,3β,6α,23-Tetrahydroxy-12-oleanen-28-oic acid
75) 2β,3β,6β,23-Tetrahydroxy-12-oleanen-28-oic acid
76) 2β,3β,16β,21β-Tetrahydroxy-12-oleanen-28-oic acid
77) 2β,3β,16α,23-Tetrahydroxy-12-oleanen-28-oic acid
78) 2α,3β,19α,23-Tetrahydroxy-12-oleanen-28-oic acid
79) 2α,3β,19β,23-Tetrahydroxy-12-oleanen-28-oic acid
80) 2α,3β,19α,24-Tetrahydroxy-12-oleanen-28-oic acid
81) 2α,3β,21β,23-Tetrahydroxy-12-oleanen-28-oic acid
82) 2α,3β,23,24-Tetrahydroxy-12-oleanen-28-oic acid
83) 2β,3β,23-Trihydroxy-5,12-oleanadien-28-oic acid
84) 2α,3α,24-Trihydroxy-11,13(18)-oleanadien-28-oic acid
85) 2α,3β,13β-Trihydroxy-28-oleananoic acid
86) 2β,3β,16α-Trihydroxy-12-oleanene-23,28-dioic acid
87) 2α,3β,18β-Trihydroxy-12-oleanene-23,28-dioic acid
88) 2α,3β,19α-Trihydroxy-12-oleanene-23,28-dioic acid
89) 2α,3β,19β-Trihydroxy-12-oleanene-23,28-dioic acid
90) 2α,3β,19α-Trihydroxy-12-oleanene-24,28-dioic acid
91) 2α,3β,19β-Trihydroxy-12-oleanene-24,28-dioic acid
92) 2β,3β,23-Trihydroxy-12-oleanene-28,30-dioic acid
93) 2β,3β,27-Trihydroxy-12-oleanene-23,28-dioic acid
94) 2α,3β,18β-Trihydroxy-12-oleanen-28-oic acid
95) 2α,3β,19α-Trihydroxy-12-oleanen-28-oic acid
96) 2α,3β,19α-Trihydroxy-12-oleanen-29-oic acid
97) 2α,3β,21β-Trihydroxy-12-oleanen-28-oic acid
98) 2α,3α,23-Trihydroxy-12-oleanen-28-oic acid
99) 2α,3α,24-Trihydroxy-12-oleanen-28-oic acid
100) 2α,3β,30-Trihydroxy-12-oleanen-28-oic acid

The compounds listed above are preferably derived from banaba leaves (*Lagerstroemia speciosa Linn.* or *Pers.),* loquat, soapberry or the like. Triterpenes represented by general formula (1) or (2) are preferably obtained from banaba leaves by extracting banaba leaves with alcohol or the like to prepare banaba extract (with further concentration to prepare a banaba extract concentrate if necessary), and purifying the extract.

The extraction is preferably carried out using fresh or dried banaba leaves, and the fresh leaves may be dried for example by natural drying, air drying or forced drying. The drying is preferably carried out to a "toasted dry" state with a moisture content of no greater than 20 wt% and preferably no greater than 10 wt% in order to prevent growth of microorganisms and ensure storage stability. The dried banaba leaves may be extracted directly, but they may instead be extracted after pulverization and chopping.

The banaba leaves prepared in the manner described above may be extracted to obtain banaba extract, using an extraction solvent such as hot water or an alcohol such as methanol or ethanol. In this case the conditions employed are preferably such as to yield a fixed content of the above-mentioned triterpenes in the extract. Examples of extraction methods include the following methods 1 to 3.

Method 1: Ethanol or an aqueous ethanol solution (50-80 wt% ethanol content) is added to dried pulverized banaba leaves (raw material) at 5-20 times by weight and preferably 8-10 times by weight with respect to the raw material, and the mixture is heated to reflux for extraction at a temperature from normal temperature to 90°C and preferably from about 50°C to 85°C, for a period from 30 minutes to 2 hours. The extraction is repeated 2 or 3 times.

Method 2: Methanol or an aqueous methanol solution (50-90 wt% methanol content) is added at 3-20 times by weight to dried pulverized banaba leaves, and the mixture is heated to reflux for extraction in the same manner as in Method 1. The extraction procedure is preferably carried out at a temperature from normal temperature to 65°C for a period from 30 minutes to 2 hours. The extraction procedure may be carried out once or repeated two or more times.

Method 3: Hot water is added at 3-20 times by weight to dried pulverized banaba leaves, and the mixture is heated to reflux for extraction at a temperature of 50-90°C and preferably 60-85°C, for a period from 30 minutes to 2 hours.

The above Methods 1 to 3 for preparing banaba extract may be combined as required. For example, Method 1 and Method 2 can be combined together. Of these methods, Methods 1 and 2 are preferred, but Method 1 is particularly preferred.

To facilitate handling, banaba extract is usually processed into banaba concentrate by concentration and drying. Regarding post-extraction concentration and drying, if the concentrate is stored at high temperature for a long time, the active components can deteriorate, so this is preferably performed in a relatively short time. For this purpose, it is advantageous to perform the concentration and drying under reduced pressure. The extract obtained as described above is filtered, and then concentrated under reduced pressure at a temperature of 60°C or less. The obtained solid is dried at a temperature of 50-70°C under reduced pressure (a higher reduced pressure than during the concentration). The dried solid is then crushed to obtain a powdered concentrate. Banaba extract concentrate is not limited to the powder form, and may be processed into tablets or granules. The banaba extract concentrate obtained by these methods contains corosolic acid, banaba polyphenols and other active components.

The triterpenes represented by general formula (1) or (2) are purified from the banaba extract or banaba extract concentrate obtained in the manner described above, and the purification may be accomplished by a publicly known method. For example, the following method may be employed for purification of corosolic acid from banaba extract.

Specifically, after suspending the banaba extract in water, it is distributed in ether or hexane to first remove low polarity components. The aqueous layer is then successively eluted with water, methanol and acetone using Diaion HP-20 column chromatography or the like. The methanol fraction containing corosolic acid is then subjected to separation and purification by silica gel column chromatography and high performance liquid chromatography so as to isolate the corosolic acid. Purification is easier if low polarity components are removed by ether or hexane and separation is performed using Diaion HP-20 column chromatography or the like (particularly if the extract amount is large), but this is not absolutely necessary, and the extract may be directly separated by silica gel column chromatography and then finally purified by high performance liquid chromatography.

The triterpenes isolated and purified from the banaba extract or banaba extract concentrate may be used directly, or they may also be acylated (for example, acetylated), or subsequently deacylated (for example, deacetylated). In the case of corosolic acid, for example, it is preferably acylated (for example, acetylated) and then deacylated. By acylating (for example, acetylating) and then deacylating the corosolic acid, it is possible to obtain corosolic acid of very high purity (approximately 100%).

Acetylation of the corosolic acid may be carried out, for example, by first dissolving the corosolic acid isolated and purified from banaba extract in anhydrous pyridine, adding acetic anhydride and allowing the mixture to stand at room temperature for about 12 hours, and then adding ice water to the reaction mixture and performing extraction several times (about 3 times) with chloroform. Next, the chloroform layer may be dewatered with sodium sulfate, the sodium sulfate removed by filtration, and then the chloroform distilled off under reduced pressure and recrystallization performed from hexane to obtain acetylcorosolic acid. As an example of a method of deacylating the acylated corosolic acid there may be mentioned a method wherein hydrolysis is performed with an alkali such as potassium hydroxide or sodium hydroxide.

### Examples

The present invention will now be explained in greater detail through examples and comparative examples, with the understanding that these examples are in no way limitative on the invention.

(Example 1)
The following test was conducted to confirm that corosolic acid has an early insulin secretion stimulating effect. A glucose tolerance test was performed after administration of corosolic acid or a placebo, in a double-blind crossover manner, and the blood insulin level (IRI: immunoreactive insulin) was assayed. Significant change in the IRI was judged by Student's *t*-test with the significance level (p value) as 0.05.

First, 31 borderline diabetic patients as test subjects were orally administered corosolic acid (10 mg, ≥99% purity) or a placebo, and blood was sampled immediately thereafter with the blood insulin level at that time designated as the value at 0 minutes. Immediately after blood sampling, 75 g of glucose was orally administered to each test subject to start a glucose tolerance test, and blood was sampled after periods of 30 minutes, 60 minutes, 90 minutes, 120 minutes and 180 minutes for measurement of the blood insulin level.

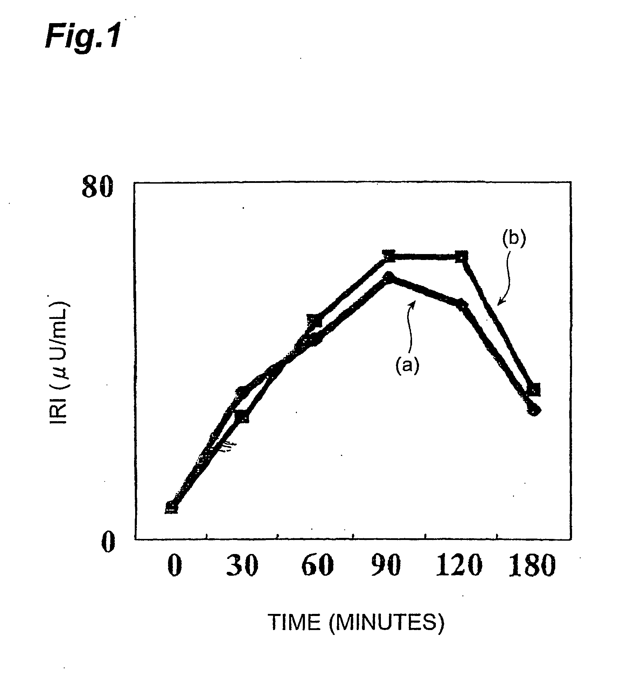

Fig. 1 is a graph showing changes in the blood insulin levels during the glucose tolerance test. The horizontal axis represents time (minutes) after start of the glucose tolerance test, and the vertical axis represents IRI (µU/mL). In the graph, (a) is a case with administration of corosolic acid and (b) is a case with administration of the placebo.

As seen by the results in Fig. 1, administration of corosolic acid produced a significant increase in blood insulin level at 30 minutes after start of the glucose tolerance test, compared to administration of the placebo. However, a significant drop in blood insulin level was seen at 120 minutes after start of the glucose tolerance test.

This demonstrated that corosolic acid stimulates early insulin secretion. It was also shown that no excess insulin is secreted in the absence of blood glucose increase. It was further suggested that corosolic acid is an early insulin secretion stimulator which is glucose-dependent.

(Example 2)
KK-Ay mice (CLEA Japan, Inc.), a genetic type II diabetes model, were used as the test animals. The KK-Ay mice used were 8 weeks old and had a blood glucose level of 300 mg/100 mL. The animal feeding conditions were at constant temperature (22 ± 2°C) throughout the test period, and the light-dark period was 12 hours (light period: 9:00 am - 9:00 pm). The feed (CE-2, CLEA Japan, Inc.) and water (tap water) were made freely available. The test substance was forcefully administered to the KK-Ay mice using a glass syringe and oral probe. The dose of each test substance was 10 mg/kg. Blood was sampled from the ocular fundus, immediately prior to and at 4 hours and 7 hours after the administration (for tormentic acid, immediately prior to and at 4 hours after the administration). The blood glucose was measured with a Glucose CII Test Wako (Wako Pure Chemical Industries Co., Ltd.).

The test substances used for this example were:
corosolic acid (isolated from banaba leaves),
maslinic acid (isolated from banaba leaves),
asiatic acid (purchased from LKT Laboratories),
2α,19α-dihydroxy-3-oxo-urs-12-en-28-oic acid (isolated from loquat callus),
ursolic acid (purchased from LKT Laboratories),
oleanolic acid (purchased from LKT Laboratories),
α-amyrin (purchased from LKT Laboratories),
β-amyrin (purchased from LKT Laboratories),
hederagenin (purchased from LKT Laboratories),
18β-glycyrrhetinic acid (purchased from Wako Pure Chemical Industries Co., Ltd.),
sapindoside B (isolated from soapberry), and
tormentic acid (isolated from loquat callus).

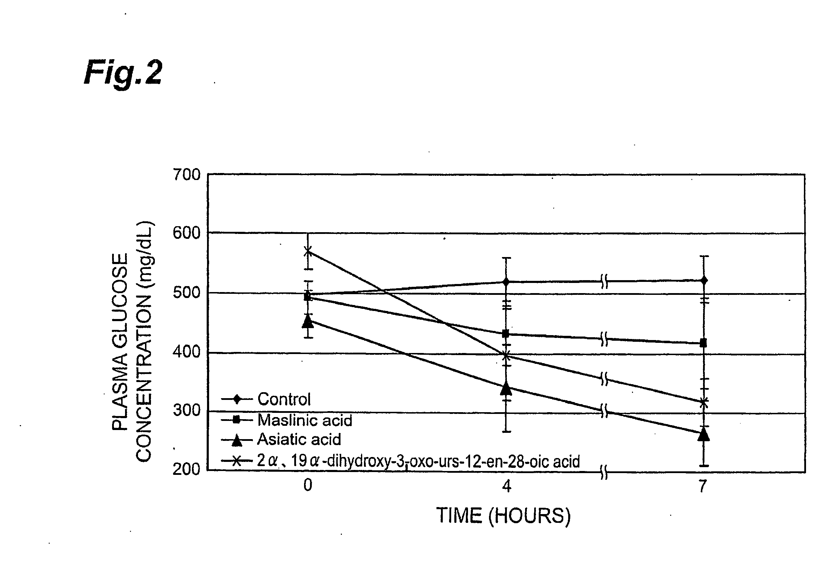

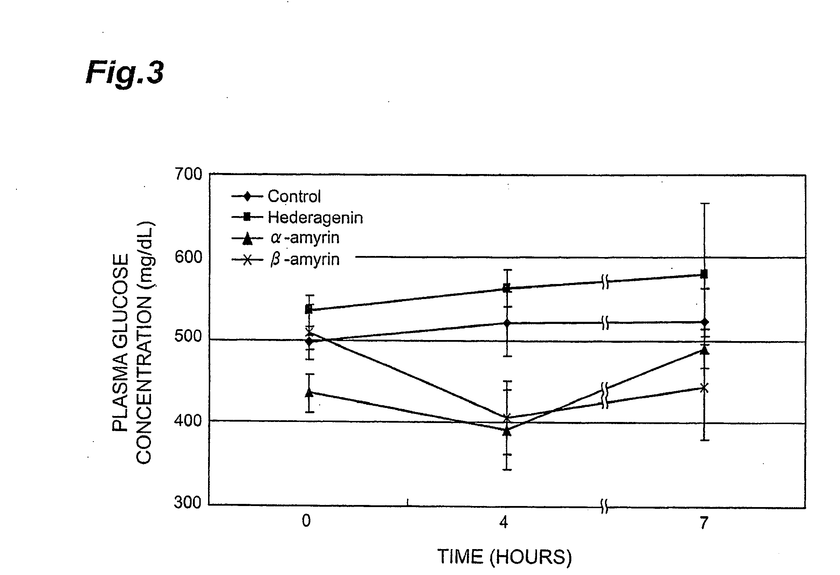

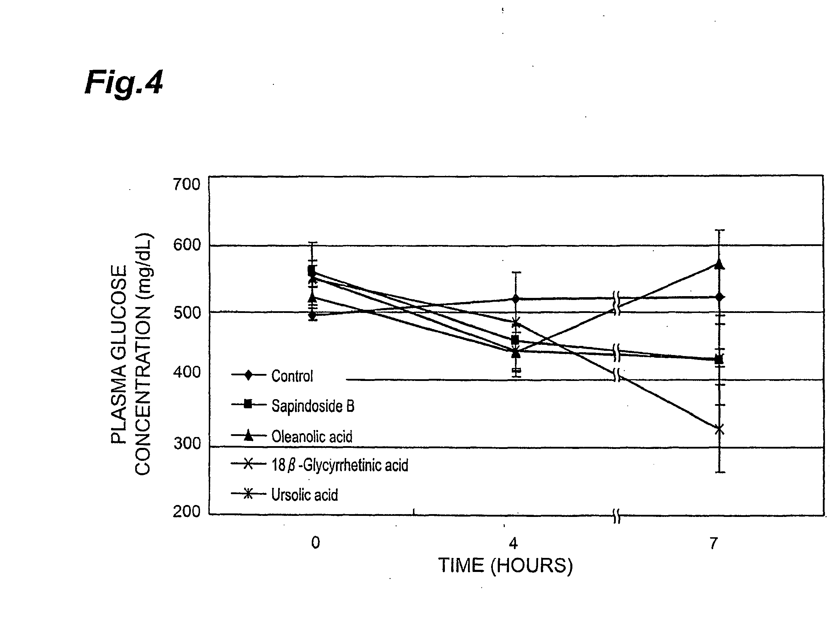

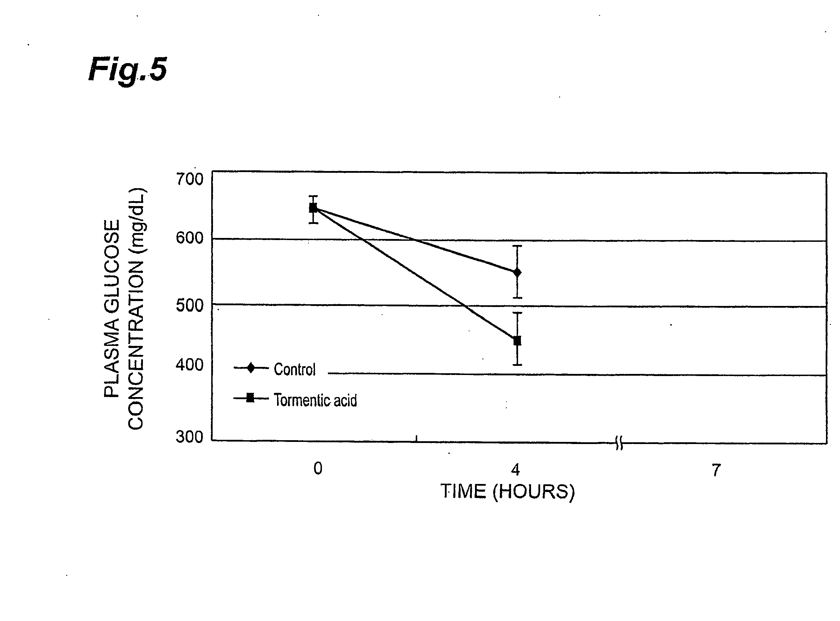

The blood glucose levels were measured using four KK-Ay mice for each test substance. The measurement results were recorded as mean ± SE. Significant change in the blood glucose level was judged by Student's *t*-test with the significance level (p value) as 0.05. The changes in blood glucose levels are shown in Figs. 2 to 5.

As seen in Figs. 2 to 5, corosolic acid, asiatic acid, 2α,19α-dihydroxy-3-oxo-urs-12-en-28-oic acid and tormentic acid, which are triterpenes represented by general formula (1) or (2), exhibited significant hypoglycemic effects. Maslinic acid did not exhibit a significant hypoglycemic effect but did exhibit a tendency toward reducing blood glucose level. On the other hand, ursolic acid, oleanolic acid, α-amyrin, β-amyrin, hederagenin, 18β-glycyrrhetinic acid and sapindoside B, which are not triterpenes represented by general formula (1) or (2), exhibited no definite hypoglycemic effect.

Based on Example 1, the hypoglycemic effects of corosolic acid, asiatic acid, 2α,19α-dihydroxy-3-oxo-urs-12-en-28-oic acid, tonnentic acid and maslinic acid, which are triterpenes represented by general formula (1) or (2), can all be attributed to early insulin secretion stimulating effects which are glucose-dependent.

### Industrial Applicability

The early insulin secretion stimulator of the present invention can be used as a drug for the treatment and prevention of diabetes mellitus.

## Claims

1. An early insulin secretion stimulator consisting of a triterpene represented by the following general formula (1) and/or a triterpene represented by the following general formula (2). 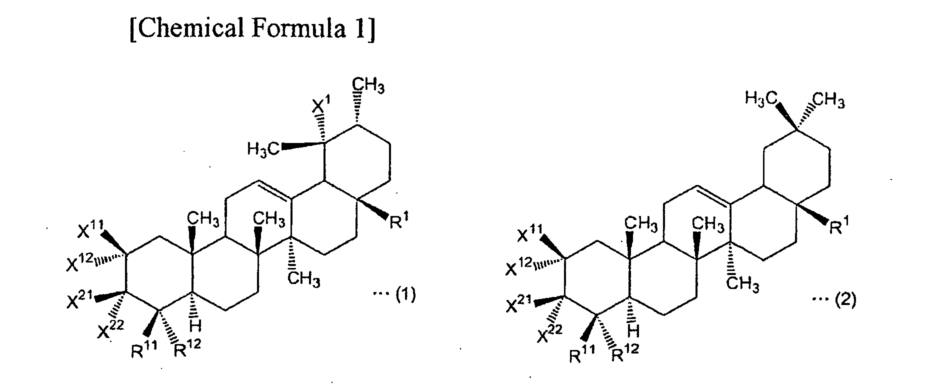 [where R¹ represents COOH, CHO, CH₃ or CH₂OH, R¹¹ and R¹² each independently represent CH₃, CH₂OH or COOH, X¹ represents hydrogen or OH, and X¹¹, X¹², X²¹ and X²² each independently represent hydrogen, OH or acyl, with the proviso that two among X¹¹, X¹², X²¹ and X²² are hydrogen and the other two are OH or acyl, X¹¹ and X¹² are not simultaneously OH or acyl, and X¹¹ and X¹² or X²¹ and X²² may together form an =O group.]

2. The early insulin secretion stimulator according to claim 1, wherein R¹ is COOH.

3. The early insulin secretion stimulator according to claim 1 or 2, wherein at least one of R¹¹ and R¹² is CH₂OH.

4. The early insulin secretion stimulator according to any one of claims 1 to 3, wherein X¹ is OH.

5. The early insulin secretion stimulator according to any one of claims 1 to 4, wherein at least one OH group of X¹¹, X¹², X²¹ and X²² is esterified.

6. The early insulin secretion stimulator according to any one of claims 1 to 4, wherein at least one OH group of X¹¹, X¹², X²¹ and X²² is etherified.

7. The early insulin secretion stimulator according to any one of claims 1 to 4, wherein two from among X¹¹, X¹², X²¹ and X²² are the same acyl group.

8. The early insulin secretion stimulator according to any one of claims 1 to 7, wherein said acyl group is a group represented by R²CO-(with the proviso that R² is an alkyl group having 1-17 carbon atoms or a substituted or unsubstituted aryl group).

9. The early insulin secretion stimulator according to any one of claims 1 to 8, wherein said acyl group is acetyl.

10. The early insulin secretion stimulator according to any one of claims 1 to 9, which is glucose-dependent.
